# EUROPÄISCHE PATENTSCHRIFT

(11) **EP 1 523 571 B1**
(45) Veröffentlichungstag und Bekanntmachung des Hinweises auf die Patenterteilung: **09.07.2008**
(21) Anmeldenummer: 03732526.3
(22) Anmeldetag: 04.06.2003
(51) Int. Cl.: C12Q 1/48, C12Q 1/68, A61P 3/08, C07K 16/00, A01K 67/027

(54) **SGK UND NEDD ALS DIAGNOSTISCHE UND THERAPEUTISCHE TARGETS**
SGK AND NEDD USED AS DIAGNOSTIC AND THERAPEUTIC TARGETS
SGK ET NEDD UTILISES COMME CIBLES DIAGNOSTIQUES ET THERAPEUTIQUES

(30) Priorität: 04.06.2002 DE 10225844
(43) Veröffentlichungstag der Anmeldung: 20.04.2005
(73) Patentinhaber: Lang, Florian Physiologisches Institut I, 72076 Tübingen (DE)
(72) Erfinder: LANG, Florian, Physiologisches Institut I, 72076 Tübingen (DE); DIETER, Michael, 72770 Reutlingen (DE); LANG, Karl, 72076 Tübingen (DE)
(74) Vertreter: Patentanwälte Ruff, Wilhelm, Beier, Dauster & Partner
(86) Internationale Anmeldenummer: PCT/EP2003/005847
(87) Internationale Veröffentlichungsnummer: WO 2003/102206

(56) Entgegenhaltungen:
- WO-A-00/18918
- WO-A-00/62781
- WO-A-89/11295
- WO-A-99/64062
- DE-C- 4 218 669
- OKU A ET AL: "T-1095, AN INHIBITOR OF RENAL NA+-GLUCOSE COTRANSPORTERS, MAY PROVIDE A NOVEL APPROACH TO TREATING DIABETES" DIABETES, NEW YORK, NY, US, vol. 48, September 1999 (1999-09), pages 1794-1800, XP002950525 ISSN: 0012-1797
- BARFULL ANNA ET AL: "Regulation of SGLT1 expression in response to Na(+) intake." AMERICAN JOURNAL OF PHYSIOLOGY. REGULATORY, INTEGRATIVE AND COMPARATIVE PHYSIOLOGY. UNITED STATES MAR 2002, vol. 282, no. 3, March 2002 (2002-03), pages R738-R743, XP002267641 ISSN: 0363-6119
- "The Merck Index, 13th Edition" 2001, MERCK & CO., INC. , WHITEHOUSE STATION, NJ , XP002267642 "Insulin" page 895
- BUSJAHN A ET AL: "SERUM- AND GLUCOCORTICOID-REGULATED KINASE (SGK1) GENE AND BLOOD PRESSURE" HYPERTENSION, XX, XX, vol. 40, no. 3, September 2002 (2002-09), pages 256-260, XP008016812 ISSN: 0194-911X cited in the application

## Beschreibung

Die vorliegende Erfindung betrifft ein Verfahren zur in vitro-Diagnose von Prädispositionen für Fettsucht, ein Diagnosekit und Antikörper.

Der intestinale und renale Transport von Glucose wird durch den Na⁺-gekoppelten Transporter Sglt1 (sodium glucose transporter) in der apikalen Membran der Epithelzellen bewerkstelligt. Ist dieser Glucosetransport gestört, kann es zu verschiedenen Erkrankungen wie Fettsucht oder Diabetes mellitus kommen.

Bisher ist wenig über die Regulation des Sglt1 bekannt. Unlängst wurde ein neuartiger Mechanismus aufgedeckt, welcher den renalen epithelialen Na⁺-Kanal ENaC reguliert: der Kanal wird durch die Ubiquitinligase Nedd4-2 ubiquitiniert und damit für Internalisierung und Abbau vorbereitet [Debonneville C, Flores SY, Kamynina E, Plant PJ, Tauxe C, Thomas MA, Munster C, Chraibi A, Pratt JH, Horisberger JD, Pearce D, Loffing J, Staub O. Phosphorylation of Nedd4-2 by Sgk1 regulates epithelial Na(+) channel cell surface expression. EMBO J. 2001; 20: 7052-7059]. Nedd4-2 wird durch die Serum- und Glucocorticoidinduzierbare Kinase 1 (Sgk1) phosphoryliert und damit inaktiviert. Damit ist Sgk1 ein potenter Stimulator des renalen epithelialen Na⁺-Kanales [De la Rosa et al., J Biol Chem 1999;274:37834-37839 ; Boehmer et al., Cell Physiol Biochem 2000;10:187-194; Chen et al., Proc Natl Acad Sci USA 1999;96:2514-2519; Náray-Fejes-Tóth et al., J Biol Chem 1999;274:16973-16978; Lang et al., Proc Natl Acad Sci USA 2000;97:8157-8162; Chigaev et al., Am J. Physiol Renal Physiol 2001;280:F1030-F1036; Wagner et al., Cell Physiol Biochem 2001;11:209-218].

Kürzlich wurde mit einer Zwillingsstudie gezeigt, dass bestimmte "Single Nucleotide Polymorphisms" (SNP) im sgk1-Gen (E8CC/CT;I6CC) mit erhöhtem Blutdruck assoziiert sind [Busjahn A, Aydin A, Uhlmann R. et al., Serum- and glucocorticoid-regulated kinase (SGK1) gene and blood pressure. Hypertension 2002; 40:256-260].

Allgemein sind Kinasen Proteine, die eine Phosphatgruppe auf individuelle Substrate übertragen. Die Serum- und Glucocorticoid-abhängige Kinase (Sgk) wurde ursprünglich aus Rattenmammakarzinomzellen kloniert [Webster MK, Goya L, Firestone GL, Y. Biol. Chem. 268 (16): 11482-11485, 1993; Webster MK, Goya L, Ge Y, Maiyar AC, Firestone GL, Mol. Cell. Biol. 13 (4): 2031-2040, 1993].

Sgk1 wurde ursprünglich als Glucocorticoid-sensitives Gen kloniert [Webster MK, Goya L, Ge Y, Maiyar AC, Firestone GL: Characterization of Sgk, a novel member of the serine/threonine protein kinase gene family which is transcriptionally induced by glucocorticoids and serum. Mol Cell Biol 1993; 13: 2031-2040]. Eine Reihe von Untersuchungen deckten auf, daß die Sgk1 unter dem Einfluß einer Vielzahl von Stimuli steht [Lang F, Cohen P. Regulation and physiological roles of serum- and glucocorticoid-induced protein kinase isoforms. Science STKE. 2001 Nov 13; 2001 (108): RE17], wie unter anderem der Mineralcorticoide [Chen SY, Bhargava A, Mastroberardino L, Meijer OC, Wang J, Buse P, Firestone GL, Verrey F, Pearce D: Epithelial sodium channel regulated by aldosterone-induced protein Sgk. Proc Natl Acad Sci USA 1999; 96: 2514-2519; Náray-Fejes-Tóth A, Canessa C, Cleaveland ES, Aldrich G, Fejes-Tóth G: Sgk is an aldosterone-induced kinase in the renal collecting duct. Effects on epithelial Na+ channels. J Biol Chem 1999; 274: 16973-16978; Park J, Leong ML, Buse P, Maiyar AC, Firestone GL, Hemmings BA:Serum and glucocorticoid-inducible kinase (Sgk) is a target of the Pl 3-kinase-stimulated signaling pathway. EMBO J 1999; 18: 3024-3033; Brenan FE, Fuller PJ. Rapid upregulation of serum and glucocorticoid-regulated kinase (Sgk) gene expression by corticosteroids in vivo. Mol Cell Endocrinol. 2000; 30: 166: 129-36; Cowling RT, Birnboim HC. Expression of serum- and glucocorticoid-regulated kinase (Sgk) mRNA is up-regulated by GM-CSF and other proinflammatory mediators in human granulocytes. J Leukoc Biol. 2000; 67: 240-248]. Sgk1 wird durch Insulin Like Growth Factor IGF1, durch Insulin und oxidativen Streß über eine Signalkaskade durch Phosphoinositol-3-Kinase (PI3-Kinase) und Phosphoinositol-abhängige Kinase (Pdk1) stimuliert [Kobayashi T, Cohen P: Activation of serum- and glucocorticoid-regulated protein kinase by agonists that activate phosphatidylinositide 3-kinase is mediated by 3-phosphoinositide-dependent protein kinase-1 (Pdk1) and pdk2. Biochem J 1999; 339: 319-328; Park J, Leong ML, Buse P, Maiyar AC, Firestone GL, Hemmings BA: Serum and glucocorticoid-inducible kinase (Sgk) is a target of the PI 3-kinase-stimulated signaling pathway. EMBO J 1999; 18: 3024-3033; Kobayashi T, Deak M, Morrice N, Cohen P. Characterization of the structure and regulation of two novel isoforms of serum- and glucocorticoid-induced protein kinase. Biochem. J. 1999; 344: 189-197]. Die Aktivierung der Sgk1 durch Pdk1 involviert eine Phosphorylierung am Serin der Position 422. Mutation dieses Serins in ein Aspartat (^{S422D}Sgk1) führt zu einer konstitutiv aktiven Kinase [Kobayashi T, Cohen P: Activation of serum- and glucocorticoid-regulated protein kinase by agonists that activate phosphatidylinositide 3-kinase is mediated by 3-phosphoinositide-dependent protein kinase-1 (Pdk1) and pdk2. Biochem J 1999; 339: 319-328].

Später wurden zwei Isoformen der Sgk1, die Sgk2 und Sgk3 kloniert [Kobayashi T, Deak M, Morrice N, and Cohen P. 1999. Characterization of the structure and regulation of two novel isoforms of serum- and glucocorticoid-induced protein kinase. Biochem J. 344:189-197]. Alle drei Sgk-Isoformen sowie die Proteinkinase B (PKB) werden über P13-Kinase und Pdk1 aktiviert [Kobayashi, T., and Cohen, P. 1999. Activation of serum- and glucocorticoid-regulated protein kinase by agonists that activate phosphatidylinositide 3-kinase is mediated by 3-phosphoinositide-dependent protein kinase-1 (PDK1) and PDK2. Biochem J. 339:319-328].

Ein Verfahren zur Behandlung oder Prophylaxe von Fettsucht ist aus der WO 00/18918 A2 bekannt. Das Verfahren beruht unter anderem auf einer Beeinflussung der Aktivität von Sglt2 (sodium dependent glucose cotransporter) über geeignete Antikörper oder Antisenseoligonucleotide.

Ziel der Erfindung ist es, neue diagnostische Anwendungen für die Regulation der Glucoseaufnahme bereitzustellen.

Überraschenderweise konnte in Zwei-Elektroden-Spannungsklemme-Versuchen gezeigt werden, daß Nedd4-2 auch den renalen und intestinalen Na⁺-Glucosetransporter Sglt inaktiviert und daß diese Wirkung durch die Sgk1 und/oder Sgk3 und/oder PKB unterbunden wird. Da eine beschleunigte Glucoseabsorption beispielsweise die Entwicklung einer Fettsucht begünstigt, ergibt sich daraus, daß Nedd4-2, Sgk1, Sgk3 und PKB bei der Entwicklung einer Fettsucht eine kausale Rolle spielen. Durch Nachweis von Nedd4-2 und/oder Sgk1 und/oder Sgk3 und/oder PKB kann beispielsweise die Ursache der Fettsucht identifiziert und durch adäquate therapeutische und prophylaktische Maßnahmen behandelt bzw. verhindert werden. Die Fettsucht und auch die Hyperglykämie, welche durch beschleunigte intestinale Glucoseabsorption ausgelöst werden, begünstigen auch die Entwicklung eines Diabetes mellitus. Schließlich würde eine gleichzeitige Dysregulation der renalen Na⁺-Kanäle die Entwicklung einer Hypertonie nach sich ziehen. Fettsucht, Hypertonie und die Entwicklung eines Diabetes mellitus sind Schlüsselbefunde des sogenannten metabolischen Syndroms.

Umgekehrt ergibt sich, daß eine Inhibierung von Sgk1 und/oder Sgk3 und/oder PKB wiederum zu einer Hemmung des renalen und intestinalen Na⁺-Glucosetransporters Sglt führt.

Demzufolge wird die erfindungsgemäße Aufgabe durch den Gegenstand der unabhängigen Ansprüche 1 bis 5 gelöst. Eine bevorzugte Ausführung des unabhängigen Anspruchs 5 ist im Anspruch 6 genannt. Der Wortlaut sämtlicher Ansprüche wird hiermit durch Bezugnahme zum Inhalt der Beschreibung gemacht.

Es wird mindestens eine Substanz zum Nachweis der Expression und/oder Funktion von aktivierter Sgk1 und/oder aktivierter Sgk3, und/oder aktivierter PKB und/oder inaktiver Nedd4-2, offenbart. Damit ist insbesondere auch eine Diagnose von Fettsucht möglich. Vorzugsweise handelt es sich bei der Substanz mindestens um eine aus der Gruppe von Antikörpern und/oder Nukleotiden. Beispielsweise kann es sich um einen Antikörper handeln, der gegen Sgk1, Sgk3, PKB und/oder Nedd4-2 gerichtet ist, und in einem dem Fachmann bekannten Nachweisverfahren, wie z. B. ELISA (Enzyme-Linked Immunosorbent Assay) eingesetzt werden kann. Bei solchen Immunoassays wird der gegen das zu bestimmende Antigen (z.B. Sgk1, Sgk3 und/oder PKB) gerichtete spezifische Antikörper (bzw. bei Antikörperbestimmungen homologe Testantigene) an eine Trägersubstanz (z. B. Cellulose, Polystyrol) gebunden, an der sich nach der Inkubation mit der Probe Immunkomplexe bilden. In einem nachfolgenden Schritt wird diesen Immunkomplexen ein markierter Antikörper zugeführt. Durch Zugabe eines chromogenen Substrates zum Reaktionsansatz können die Immunokomplex-gebundenen Enzym-Substrat komplexe sichtbar gemacht bzw. die Antigenkonzentration in der Probe über eine photometrische Bestimmung der Immunkomplex-gebundenen Markerenzyme durch Vergleich mit Standards bekannter Enzymaktivität ermittelt werden. Wie bereits oben erwähnt, können auch Nukleotide, insbesondere Oligonukleotide, für den diagnostischen Nachweis verwendet werden, die beispielsweise mit Hilfe der sogenannten Polymerase-Kettenreaktion geeignet sind, über ein molekulargenetisches Verfahren, bei dem selektiv bestimmte DNA-Abschnitte amplifiziert werden, einen quantitativen Nachweis von beispielsweise Sgk1 zu erbringen.

Vorzugsweise werden Antikörper gegen mindestens eine phosphorylierte und/oder nicht phosphorylierte Sgk1 Konsensussequenz im Nedd 4-2- Protein eingesetzt. Unter Konsensussequenz sind hierbei die Aminosäuresequenzen zu verstehen, die die Substratstelle der Kinasen bilden, also den oder die Orte der Phosphorylierung.

Auch ist es möglich, daß inaktivierende Mutationen im Nedd4-2-Protein, insbesondere in der Sgk1 Konsensussequenz (z. B. ^{S338D}Nedd4-2 oder ^{S444D}Nedd4-2), detektiert werden. Ferner wird eine aktivierende Mutation, beispielsweise ^{S422D}Sgk1 und/oder ^{T308D,S473D}PKB, in der DNA der Patienten detektiert. Bei einer weiteren Verwendung werden entsprechende Mutationen in der RNA der Patienten detektiert. Schließlich werden entsprechende Mutationen im Sgk-, insbesondere Sgk1- und/oder Sgk3-, PKB- und/oder im Nedd-Protein, insbesondere im Nedd4-2-Protein der Patienten detektiert. Für diese Nachweise werden vorzugsweise entweder geeignete Antikörper und/oder geeignete Nukleotide, insbesondere Oligonukleotide, als Sonden eingesetzt.

Bei den zu diagnostizierenden Erkrankungen, die mit einem gestörten Glucosetransport in Verbindung stehen, handelt es sich um Fettsucht.

Die Erfindung umfasst ein Verfahren zur in vitro-Diagnose von Prädispositionen für Fettleibigkeit bzw. Fettsucht. Dieses Diagnoseverfahren ist dadurch gekennzeichnet, daß ein Polymorphismus in Sgk1 nachgewiesen wird. Es wird der Polymorphismus E8CC/CT;I6CC in Sgk1 nachgewiesen. Dieser Polymorphismus steht in unmittelbarer Korrelation mit dem Körpermassen-Index, so dass dies ein besonders geeigneter Marker ist, um Prädispositionen für Fettleibigkeit aufzuzeigen. Hierbei steht die abgekürzte Schreibweise für einen SNP in Exon 8 (C→T) und einen zweiten SNP, der sich 551 Basenpaare entfernt von der Donorstelle (Intron 6) von Exon 7 (T→C) befindet. Zum Nachweis von entsprechenden Polymorphismen wird bevorzugterweise Blut von entsprechenden Versuchstieren oder Patienten entnommen, und anhand des darin enthaltenden Genmaterials durch entsprechende Sequenzierung oder durch andere dem Fachmann geläufige Methoden die Sequenz an entsprechender Stelle bestimmt. Neben Blut eignen sich auch im Prinzip alle anderen biologischen Proben, aus denen sich genetisches Material isolieren läßt.

Weiterhin wird die Verwendung mindestens eines Wirkstoffes zur Beeinflussung des Glucosetransportes, insbesondere des intestinalen und/oder renalen Glucosetransportes offenbart. Bevorzugterweise ist für diesen Glucosetransport zumindest teilweise der Glucosetransporter Sglt, insbesondere Sglt1, verantwortlich. Durch die Beeinflussung der Expression und/oder Aktivität von Sglt, insbesondere Sglt1, kann erfindungsgemäß der Glucosetransport beeinflusst werden. Vorzugsweise wird durch den Wirkstoff eine Beeinflussung mindestens einer Sgk, insbesondere Sgk1 und/oder Sgk3, und/oder PKB und/oder eine Beeinflussung mindestens einer Nedd, insbesondere Nedd4-2, erreicht. Vorzugsweise ist der Wirkstoff gegen eine Sgk, insbesondere eine Sgk1 und/oder Sgk3, und/oder PKB und/oder eine Nedd, insbesondere Nedd4-2, gerichtet. Weiterhin ist der Wirkstoff gegen Aktivatoren, Inhibitoren, Regulatoren und/oder biologische Vorläufer einer Sgk, insbesondere von Sgk1 und/oder Sgk3, und/oder PKB und/oder einer Nedd, insbesondere von Nedd4-2, gerichtet.

Weiterhin ist der Wirkstoff ein Polynukleotid. Dieses Polynukleotid kann beispielsweise eine Antisensesequenz umfassen, welche die Expression mindestens eines der genannten Proteine vermindert oder hemmt. In einer weiteren bevorzugten Ausführungsform kodiert das Polynukleotid für ein Peptid, vorzugsweise ein Polypeptid, wobei dieses Peptid die Expression und/oder Funktion einer Sgk, insbesondere Sgk1 und/oder Sgk3, und/oder PKB und/oder einer Nedd, insbesondere Nedd4-2, beeinflusst. Weiterhin kann der Wirkstoff selbst bevorzugterweise ein Peptid bzw. ein Polypeptid sein, welches die Expression und/oder Funktion der genannten Proteine beeinflusst. Der Wirkstoff kann ein "small molecular compound", vorzugsweise ein "small molecular compound" mit einem Molekulargewicht < 1.000, sein.

Je nachdem, ob das Ziel eine Behandlung von Erkrankungen ist, die mit einem gestörten Glucosetransport in Verbindung stehen, oder ob die Erhöhung des Körpergewichts von Tieren bei der Tiermast angestrebt wird, müssen die jeweiligen Enzyme unterschiedlich beeinflußt werden. Zur Vorbeugung oder Behandlung von Erkrankungen, die im Zusammenhang mit einer gestörten Glucoseabsorption stehen, soll durch den Wirkstoff eine Inhibierung mindestens einer Sgk, insbesondere Sgk1 und/oder Sgk3, und/oder PKB und/oder eine Stimulierung mindestens einer Nedd, insbesondere Nedd4-2, erreicht werden. Da Sgk und PKB Kinasen sind, kommen insbesondere dem Fachmann bekannte Kinaseinhibitoren, wie z. B. Staurosporin und/oder Chelerythrin bzw. mindestens eine von deren Analoga in Frage. Da Nedd Ligasen sind, kommen für deren Stimulierung Ligaseaktivatoren in Frage. Bevorzugt werden diese Wirkstoffe zur Herstellung eines Medikamentes oder einer pharmazeutischen Zusammensetzung genutzt. Bei den zu behandelnden Erkrankungen handelt es sich vorzugsweise um das metabolische Syndrom, insbesondere um Fettsucht.

Soll dagegen, im Gegensatz zur oben beschriebenen Vorbeugung oder Behandlung von Erkrankungen, bei denen eine Erniedrigung des Glucosetransportes das Ziel ist, eine Steigerung des Glucosetransportes, beispielsweise zur Erhöhung des Körpergewichtes von Tieren bei der Tiermast, erreicht werden, wird durch den Wirkstoff vorzugsweise eine Stimulierung mindestens einer Sgk, insbesondere Sgk1 und/oder Sgk3, und/oder PKB und/oder eine Inhibierung mindestens einer Nedd, insbesondere Nedd4-2, erreicht. Durch eine Stimulierung von beispielsweise Sgk1 kommt es zu einer Inhibierung von beispielsweise Nedd4-2, was wiederum zu einem verzögerten Abbau des Glucosetransporters Sglt1 führt. Dadurch kommt es wiederum zu einer Steigerung des Glucosetransportes. Weiterhin ist der Wirkstoff mindestens ein Sgk- und/oder PKB-Aktivator, insbesondere ein Wachstumsfaktor, vorzugsweise IGF1 und/oder Insulin.

Weiterhin ist der Wirkstoff mindestens ein Stimulans für die Transkription von sgk1 und/oder sgk3 und/oder einem Gen für PKB, vorzugsweise mindestens ein Glucocorticoid, Mineralcorticoid, Gonadotropin und/oder Cytokin, insbesondere TGFβ.

Die Erfindung betrifft weiterhin einen Diagnosekit. Dieser umfaßt mindestens eine Substanz zum Nachweis der Expression und/oder Funktion von aktivierter Sgk1 und/oder aktivierter Sgk3, und/oder aktivierter PKB und/oder inaktiver Nedd4-2 zur Diagnose von Fettsucht. Der Kit kann insbesondere Antikörper und/oder Oligonukleotide zum Nachweis der entsprechenden Proteine und/oder Nukleinsäuren enthalten. Beispielsweise können hiermit die Menge und/oder die Aktivität der verschiedenen Proteine bzw. Enzyme analysiert werden. Weiterhin können auch entsprechende Mutationen in den Genen nachgewiesen werden. Bezüglich weiterer Merkmale dieses Kits wird auf die übrige Beschreibung verwiesen.

Darüber hinaus umfaßt die Erfindung Antikörper, die gegen mindestens eine phosphorylierte Kinase-Konsenssussequenz im Nedd4-2-Protein gerichtet sind. Bei dieser Kinase-Konsenssussequenz handelt es sich um die Sequenz, die durch Sgk1 phosphoryliert wird. Mit Hilfe eines solchen Antikörpers kann analysiert werden, ob Nedd4-2 durch Sgk1 phosphoryliert und damit inaktiviert wurde. Hiermit läßt sich also der Aktivitätsstatus von Nedd4-2 untersuchen. Weiterhin umfaßt die Erfindung einen Antikörper, der gegen die entsprechende nicht phosphorylierte Kinase-Konsenssussequenz im Nedd4-2-Protein gerichtet ist. Besonders bevorzugt ist es, die beiden erfindungsgemäßen Antikörper in einer Testanordnung miteinander zu kombinieren, wodurch sehr aussagekräftige Ergebnisse bezüglich des Aktivitätsstatus von Nedd4-2 erzielt werden können.

Weiterhin umfaßt die Erfindung Antikörper, die gegen mindestens eine mutierte Kinase-Konsensussequenz im Nedd4-2-Protein gerichtet sind. Hierbei handelt es sich wiederum um die Sgk1-Konsensussequenz, die entsprechend mutiert ist. Besonders bevorzugte Mutanten sind hierbei ^{S338D}Nedd4-2 und/oder ^{S444D}Nedd4-2. Entsprechende Mutationen bewirken, daß Nedd4-2 nicht mehr durch Sgk1 phosphoryliert werden kann. Ein solche Antikörper kann als hilfreiches Werkzeug eingesetzt werden, um entsprechende Mutanten zu untersuchen.

Die Herstellung der erfindungsgemäßen Antikörper erfolgt mit dem Fachmann geläufigen Methoden. Insbesondere können polyklonale oder monoklonale Antikörper hergestellt werden, wobei monoklonale Antikörper wegen ihrer im allgemeinen höheren Spezifität bevorzugt sind.

Besonders vorteilhaft können die beschriebenen Antikörper in dem erfindungsgemäßen Diagnosekit verwendet werden. Weiterhin sind die beschriebenen Antikörper auch mit großem Vorteil in der Verwendung zum Nachweis der Expression und/oder Funktion von Sgk, PKB und/oder Nedd einsetzbar. Hierbei können die Antikörper gemäß üblichen immunologischen Verfahren verwendet werden. Insbesondere können mit diesen Antikörpern die bereits erwähnten ELISA durchgeführt werden.

Es wird ferner eine Zusammensetzung, vorzugsweise eine pharmazeutische Zusammensetzung, offenbart, die mindestens einen Wirkstoff enthält, der den Glukosetransport, insbesondere den intestinalen und/oder renalen Glukosetransport, beeinflußt, und ggf. einen pharmazeutisch akzeptablen Träger. Besonders bevorzugt beeinflußt der Wirkstoff mindestens eine Sgk und/oder PKB und/oder mindestens eine Nedd. Weiterhin beeinflußt der Wirkstoff Aktivatoren, Inhibitoren, Regulatoren und/oder biologische Vorläufer einer Sgk, insbesondere von Sgk1 und/oder Sgk3, und/oder PKB und/oder einer Nedd, insbesondere von Nedd4-2.

Mit Vorteil ist der Wirkstoff ein Polynukleotid. Dieses Polynukleotid kann eine Antissensesequenz umfassen oder bilden, die die Expression der entsprechenden Gene vermindert oder hemmt. Weiterhin kann ein entsprechendes Polynukleotid so gewählt sein, dass es die Expression des jeweiligen Gens oder der Gene durch einen sogenannten dem Fachmann bekannten Dominant-Negative-Ansatz hemmt bzw. die Funktion der entsprechenden Genprodukte einschränkt. Weiterhin kann das Polynukleotid ein Peptid, vorzugsweise ein Polypeptid, kodieren, wobei dieses Peptid die Expression und/oder Funktion einer Sgk, insbesondere Sgk1 und/oder Sgk3, und/oder PKB und/oder einer Nedd, insbesondere Nedd4-2, beeinflusst. Die entsprechenden molekularbiologischen Vorgehensweisen, die für derartige Ansätze erforderlich sind, erschließen sich dem Fachmann. Weiterhin ist der Wirkstoff das beschriebene Peptid selbst. Bevorzugterweise ist der Wirkstoff ein "small molecular compound", vorzugsweise ein "small molecular compound" mit einem Molekulargewicht < 1.000.

Insbesondere zur Behandlung von Erkrankungen, die mit einem gestörten Glukosetransport in Verbindung stehen, wird durch den Wirkstoff eine Inhibierung mindestens einer Sgk und/oder PKB und/oder eine Stimulierung mindestens einer Nedd erreicht. Besonders bevorzugt ist der Wirkstoff zur Behandlung dieser Erkrankungen mindestens ein Kinaseinhibitor, vorzugsweise Staurosporin und/oder Chelerythrin bzw. eines von deren Analoga, und/oder mindestens ein Ligaseaktivator.

Zur Steigerung des Glukosetransports, insbesondere bei der Tiermast, wird durch den Wirkstoff vorzugsweise eine Stimulierung mindestens einer Sgk und/oder PKB und/oder eine Inhibierung mindestens einer Nedd erreicht. Mit Vorteil ist der Wirkstoff zur Steigerung des Glukosetransportes ein Sgk1-Aktivator, insbesondere ein Wachstumsfaktor, vorzugsweise IGF1, und/oder Insulin. Weiterhin ist der Wirkstoff ein Stimulans für die Transkription der Sgk1 und/oder Sgk3 und/oder PKB, vorzugsweise mindestens ein Glucocorticoid, Mineralcorticoid, Gonadotropin und/oder Cytokin, insbesondere TGFβ.

Die verschiedenen beschriebenen Möglichkeiten können auch miteinander kombiniert werden.

Es wird ferner ein Verfahren zur Erzeugung von transgenen Tieren, die eine erhöhte Lipidablagerung in Fettgewebe aufweisen, offenbart. Von diesem Aspekt sind Menschen ausgeschlossen. Entsprechende Tiere sind insbesondere für die Nahrungserzeugung von großem Interesse, da sie schneller Fett ansetzen. Mit diesen Tieren kann eine Mast sehr viel schneller und effektiver durchgeführt werden. Das Verfahren zur Erzeugung derartiger Tiere ist dadurch gekennzeichnet, dass in diesen Tieren die Expression und/oder Funktion von Sglt, insbesondere Sglt1, gesteigert wird. Hierdurch wird die intestinale Absorption von Glucose beschleunigt, was zu einem schnelleren Anstieg der Glucosekonzentration im Plasma führt. Hierdurch wird vermehrt Insulin ausgeschüttet, was schließlich zu einer Stimulierung der Lipidablagerung in Fettgewebe führt.

Weiterhin wird hierfür sglt, insbesondere sglt1, im Tier zur Überexpression gebracht. Dies geschieht beispielsweise durch Einbringen von entsprechenden Genkonstrukten, insbesondere Vektoren, die entsprechend starke Promotoren tragen, die einer entsprechenden sglt-Sequenz funktional vorgeschaltet sind. Weiterhin ist es auch bevorzugt, Tiere mit entsprechend starker Expression von sglt, insbesondere sglt1, zu klonieren. Die methodischen Vorgehensweisen hierfür erschließen sich dem Fachmann.

Weiterhin wird Sgk, insbesondere Sgk1 und/oder Sgk3, und/oder PKB in der Expression und/oder Funktion gesteigert. Hierdurch wird im Endergebnis ebenfalls die Aktivität bzw. die Proteinmenge von Sglt, insbesondere Sglt1, gesteigert, so dass der Glucosetransport erhöht wird. Hierfür können die entsprechenden Gene mit geläufigen molekularbiologischen Methoden überexprimiert werden. Andererseits können auch Genkonstrukte in den Organismus eingebracht bzw. integriert werden, welche entsprechende konstitutiv aktive Mutanten exprimieren. Besonders bevorzugt sind hierbei die Mutanten ^{S422D}sgk1 und/oder ^{T308D,S473D}PKB. Entsprechende Mutanten sind in ihrer Aktivität von anderen aktivierenden Enzymen, insbesondere Kinasen, unabhängig und daher ständig aktiv. Sie hemmen den durch die Ubiquitinligase Nedd, insbesondere Nedd4-2, verursachten Abbau von Sglt, insbesondere Sglt1, so dass im Ergebnis der Glucosetransport gesteigert wird.

Weiterhin wird die Ubiquitinligase Nedd, insbesondere Nedd4-2, in ihrer Expression und/oder Funktion gemindert. Auch dies bewirkt eine Steigerung des Glucosetransports durch einen verminderten Abbau von Sglt, insbesondere Sglt1. Eine entsprechende Minderung der Expression und/oder Funktion von Nedd kann ebenfalls mit üblichen molekularbiologischen Methoden, wie beispielsweise Antisense- oder Dominant-Negative-Ansätzen, erreicht werden. Besonders bevorzugt ist es, geeignete Mutationen von nedd, insbesondere von nedd4-2, stabil in den Organismus zu integrieren oder das native Gen für Nedd auszuschalten, um so langfristig die Expression von diesem Enzym zu mindern oder zu hemmen. Entsprechende Vorgehensweisen sind dem Fachmann bekannt. Besonders bevorzugt ist es hierbei, mindestens eine inaktivierende Mutation in Nedd, insbesondere in Nedd4-2, einzufügen. Mit großem Vorteil können hierbei die Mutationen ^{S338D}nedd4-2 und/oder ^{S444D}nedd4-2 verwendet werden. Tiere, die durch das Verfahren herstellbar sind, werden ebenfalls umfaßt.

Die beschriebenen Merkmale und weitere Merkmale der Erfindung ergeben sich aus der nachfolgenden Beschreibung von bevorzugten Ausführungsformen in Verbindung mit den Unteransprüchen und den Figuren. Hierbei können die einzelnen Merkmale jeweils für sich oder zu mehreren in Kombination miteinander verwirklicht sein.

In den Abbildungen zeigen:

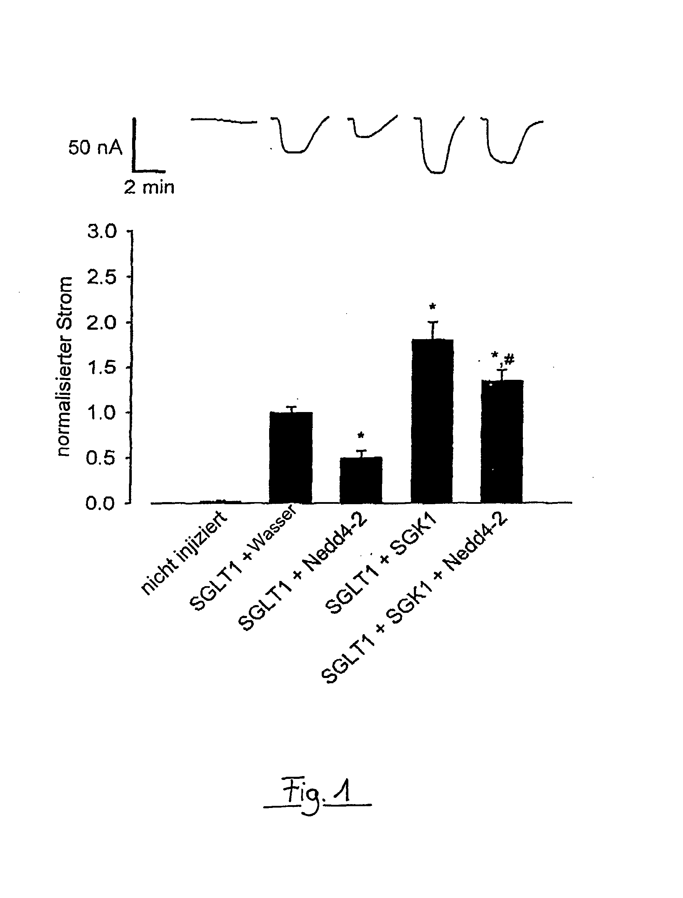

- Fig. 1:: Regulation des Na⁺-gekoppelten Glucosetransporters Sglt1 durch Nedd4-2 und Sgk1.
Oberer Teil: Originale Messdaten; unterer Teil: arithmetische Mittelwerte ± SEM (n = 6-15). *Xenopus laevis* Oocyen wurden mit cRNA von sglt1, nedd4-2 und/oder sgk1 injziert. Während Nedd4-2 die durch 20 mM Glucose induzierten Ströme, die in Oocyten, die Sglt1 exprimierten, herunterregulierte, stimulierte Sgk1 die Ströme und drehte den Effekt von Nedd4-2 um.
* zeigt die signifikanten Unterschiede zu den Strömen an, die in Oocyten gemessen wurden, die Sglt1 allein exprimierten.
# zeigt die signifikanten Unterschiede zu den entsprechenden Werten in Oocyten an, die Sglt1 und Nedd4-2 exprimierten.

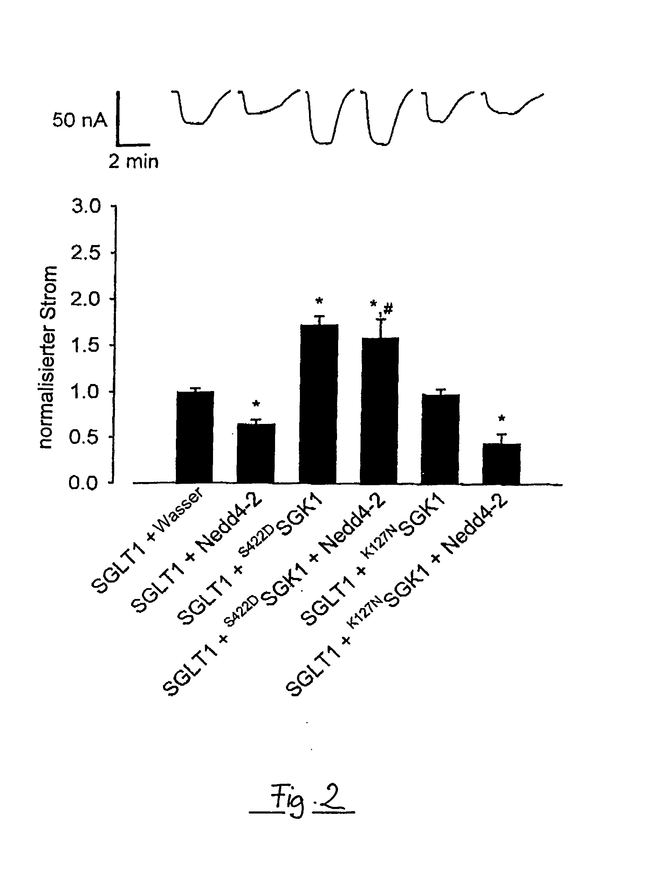

- Fig. 2: Regulation des Na⁺-gekoppelten Glucosetransporters Sglt1 durch Nedd4-2, konstitutiv aktiver ^{S422D}Sgk1 und inaktiver ^{K127N}Sgk1. Oberer Teil: Originale Messverläufe; unterer Teil: arithmetische Mittelwerte ± SEM. (n = 8-71). *Xenopus laevis* Oocyten wurden mit cRNA von sglt1, nedd4-2 und/oder ^{S422D}sgk1 oder ^{K127N}sgk1 injiziert. Während Nedd4-2 signifikant die 20 mM Glucose induzierten Ströme in Oocyten, die Sglt1 exprimierten, herunterregulierte, stimulierte ^{S422D}Sgk1, aber nicht ^{K127N}Sgk1, die Ströme und drehte den Effekt von Nedd4-2 um.
* zeigt die signifikanten Unterschiede zu den Strömen an, die in Oocyten gemessen wurden, die Sglt1 allein exprimierten.
# zeigt die signifikanten Unterschiede zu den entsprechenden Werten in Oocyten an, die Sglt1 und Nedd4-2 exprimierten.

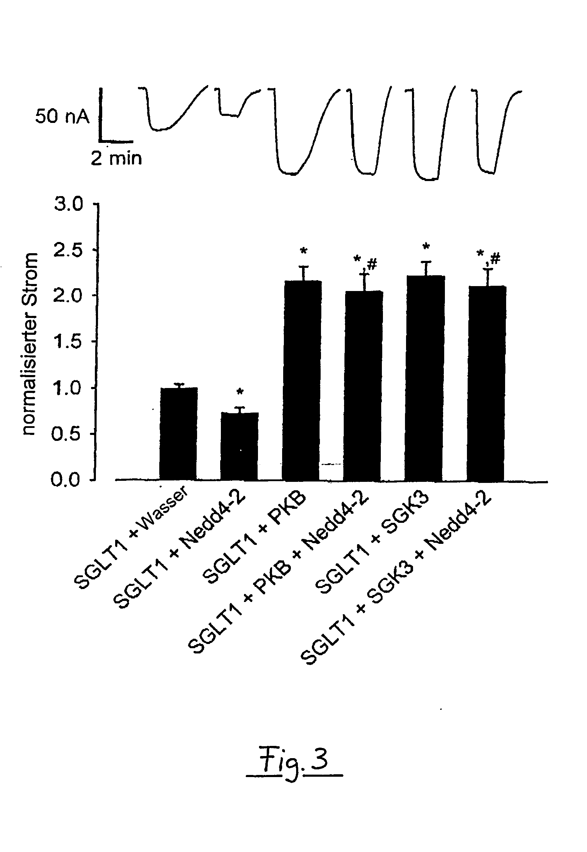

- Fig. 3: Regulation des Na⁺-gekoppelten Glucosetransporters Sglt1 durch Nedd4-2, ^{T308D,S473D}PKB und Sgk3.
Oberer Teil: Originale Messverläufe; unterer Teil: arithmetische Mittelwerte ± SEM. *Xenopus laevis* Oocyten wurden mit cRNA von sglt1, nedd4-2, ^{T308D,S473D}PKB und/oder sgk3 injiziert. Nedd4-2 regulierte signifikant die durch 20 mM Glucose induzierten Ströme in Oocyten herunter, die Sglt1 exprimierten. ^{T308D,S473D}PKB und Sgk3 stimulierten die Ströme und drehten den Effekt von Nedd4-2 um.
* zeigt die signifikanten Unterschiede zu Strömen an, die in Oocyten gemessen wurden, die Sglt1 allein exprimierten.
# zeigt die signifikanten Unterschiede zu den entsprechenden Werten in Oocyten an, die Sglt1 und Nedd4-2 exprimierten.

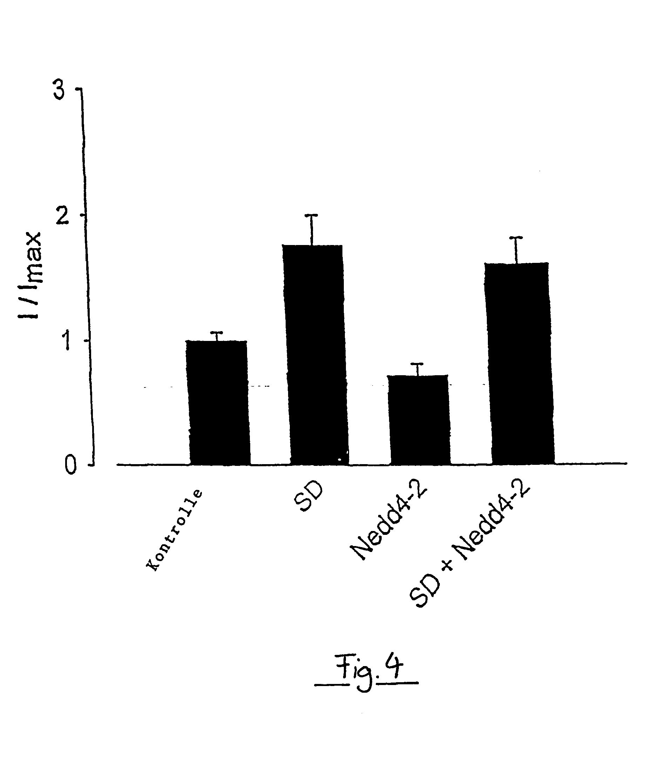

- Fig. 4:: Regulation des Na⁺-gekoppelten Glucosetransporters Sglt1 durch Nedd4-2 und Sgk1. Arithmetische Mittelwerte ± SEM (n = 18). Xenopus-Oocyten wurden mit cRNA von sglt1, nedd4-2 und/oder ^{S422D}Sgk1 (SD) injiziert. Während Koexpression von Nedd4-2 die durch Zugabe von 5 mmol Glucose induzierten Ströme herabsetzte, wurden die Ströme durch Koexpression von konstitutiv aktiver Kinase ^{S422D}sgk1 signifikant gesteigert.

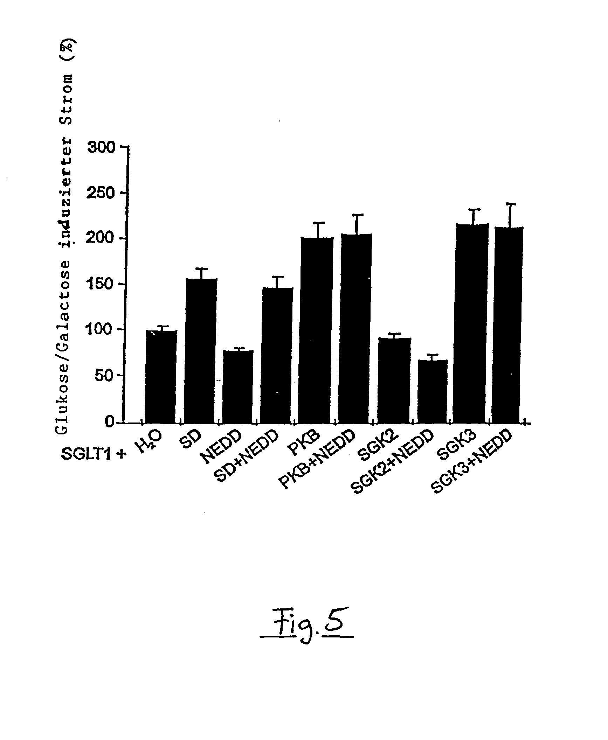

- Fig. 5:: Regulation des Na⁺ gekoppelten Glucosetransporters Sglt1 durch Nedd4-2, die Sgk3 und die PKB. Arithmetische Mittelwerte ± SEM (experimentelles Vorgehen wie bei Fig. 4).

### BEISPIEL

### Methoden

### 1. Expression in Xenopus laevis Oocyten und Zwei-Elektroden-Spannungsklemme

cRNA , die für Wildtyp Sgk1 kodiert [Waldegger S, Barth P, Raber G, Lang F: Cloning and characterization of a putative human serine/threonine protein kinase transcriptionally modified during anisotonic and isotonic alterations of cell volume. Proc Natl Acad Sci USA 1997; 94; 4440-4445], die für konstitutiv aktive Sgk1 (^{S422D}Sgk1) und für inaktive Sgk1 kodiert (^{K127N}Sgk1) [Kobayashi T, Cohen P. Activation of serum- and glucocorticoid-regulated protein kinase by agonists that activate phosphatidylinositide 3-kinase is mediated by 3-phosphoinositide-dependent protein kinase-1 (PDK1) and PDK2. Biochem J. 1999;339:319-28.], Wildtyp Sgk3 und PKB, [Kobayashi T, Deak M, Morrice N, Cohen P. Characterization of the structure and regulation of two novel isoforms of serum- and glucocorticoid-induced protein kinase. Biochem J. 1999;344:189-97], konstitutiv aktiver ^{T308D,S473D}PKB [Alessi DR, Cohen P. Mechanism of activation and function of protein kinase B. Curr Opin Genet Dev. 1998;8:55-62], von Wildtyp Nedd4-2 [Debonneville C, Flores SY, Kamynina E, Plant PJ, Tauxe C, Thomas MA, Munster C, Chraibi A, Pratt JH, Horisberger JD, Pearce D, Loffing J, Staub O. Phosphorylation of Nedd4-2 by Sgk1 regulates epithelial Na(+) channel cell surface expression. EMBO J. 2001; 20: 7052-7059] und von Wildtyp Sglt1 [Hediger MA, Coady MJ, Ikeda TS, Wright EM. Expression cloning and cDNA sequencing of the Na+/glucose co-transporter. Nature. 1987; 330: 379-381] wurden *in vitro* synthetisiert [Wagner CA, Friedrich B, Setiawan I, Lang F, Bröer S: The use of Xenopus laevis oocytes for the functional characterization of heterologously expressed membrane proteins. Cell Physiol Biochem 2000; 10: 1-12]. Dissektion der *Xenopus laevis* Ovarien, Kollektion und Behandlung der Oocyten wurden bereits detailliert beschrieben [Wagner CA, Friedrich B, Setiawan I, Lang F, Bröer S: The use of Xenopus laevis oocytes for the functional characterization of heterologously expressed membrane proteins. Cell Physiol Biochem 2000; 10: 1-12]. Die Oocyten wurden mit 5 ng von humanem sglt1, 7,5 ng humanem sgk1, ^{K127N}sgk1, ^{S422D}sgk1, sgk3, PKB oder ^{T308D,S473D}PKB und/oder mit 5 ng von *Xenopus* nedd4-2 injiziert. Kontrolloocyten wurden mit Wasser injiziert. Elektrophysiologische Experimente wurden bei Raumtemperatur 3 Tage nach Injektion der jeweiligen cRNAs durchgeführt. Die durch extrazelluläre Verabreichung von 20 mM bzw. 5 mM Glucose induzierten Ströme wurden mit der Zwei-Elektroden-Spannungsklemme [Wagner CA, Friedrich B, Setiawan I, Lang F, Bröer S: The use of Xenopus laevis oocytes for the functional characterization of heterologously expressed membrane proteins. Cell Physiol Biochem 2000; 10: 1-12] gemessen und als Maß für den Glucosetransport genommen. Die Daten wurden bei 10 Hz gefiltert und mit dem MacLab Digital to Analog Converter und entsprechender Software ausgewertet (AD Instruments, Castle Hill, Australia). Die Kontrollbadlösung (ND 96) enthielt 96 mM NaCl, 2 mM KCI, 1,8 mM CaCl₂, 1 mM MgCl₂ und 5 mM HEPES, pH 7,4. Alle Substanzen wurden in den angegebenen Konzentrationen eingesetzt. Die endgültigen Lösungen wurden mit HCl bzw. NaOH auf den angegebenen pH bzw. pH 7,4 titriert. Die Flußgeschwindigkeit der Superfusionslösung war 20 ml/min und erreichte einen kompletten Lösungswechsel innerhalb von 10 s.

Für die Berechnungen wurden die Daten als arithmetische Mittelwerte ± SEM angegeben. n ist die Zahl der untersuchten Oocyten. Alle Experimente wurden in mindestens drei verschiedenen Gruppen von Oocyten durchgeführt. In allen Wiederholungen wurden qualitativ ähnliche Daten erhalten. Die Ergebnisse wurden mit dem Student t-test auf signifikante Unterschiede getestet. Nur Ergebnisse mit P < 0,05 wurden als statistisch signifikant verwertet.

### 2. Zwillingsstudien

126 Paare von eineiigen und 70 Paare von zweieiigen Zwillingen wurden für die Studien zur Blutdruckregulation und zu kardiovaskulären Phänotypen rekrutiert. Die Eltern der zweieiigen Zwillingen wurden ebenfalls einbezogen. Alle Teilnehmer waren deutsche Kaukasier aus verschiedenen Teilen Deutschlands. Zur Bestimmung der Zygosität und anderer molekulargenetischer Studien wurde Blut von allen Zwillingen und den Eltern der zweieiigen Zwillinge entnommen. Jeder Teilnehmer unterzog sich einer medizinischen und physischen Untersuchung. Keiner der Teilnehmer hatte eine Familiengeschichte mit chronischen medizinischen Krankheiten. Ein "Single Nucleotide Polymorphism" (SNP) wurde in Exon 8 (C→T) und ein zweiter SNP 551 Basenpaare entfernt in der Donorstelle (Intron 6) von Exon 7 (T→C) identifiziert [Busjahn A, Aydin A, Uhlmann R et al., Serum- and glucocorticoid-regulated kinase (SGK1) gene and blood pressure. Hypertension 2002; 40:256-260)]. Diese beiden einzelnen SNPs, Intron 6 (T→C) und Exon 8 (C→T), wurden analysiert.

Beschreibende Statistiken für beide SNPs zeigten einen rezessiven Aktionsmodus. Daher wurde die Assoziationsanalyse auf zwei Gruppenvergleiche gestützt, und zwar auf homozygote Träger der Variante vs. heterozygote Träger und Nicht-Träger. Die Unabhängigkeit der beiden SNPs wurde mit dem chi²-Test getestet. Die Beziehung zwischen SNPs und den Phenotypen wurde durch eindimensionales ANOVA getestet, wobei beide Polymorphismen zur selben Zeit inkorporiert wurden. Diese Analyse bezog beide Teile der zweieiigen Zwillingspaare sowie ein zufällig ausgewähltes Teil der eineiigen Zwillingspaare ein. Dieser Test war zuverlässiger als der t-Test, und es konnten gleichzeitig beide Polymorphismen unter Einbezug ihrer Interaktion berücksichtigt werden. Außerdem konnte so auch die Anzahl der Untersuchungen reduziert werden. Da beide Teile der zweieiigen Zwillingspaare nicht unabhängig voneinander sind, wurden familiäre Effekte sowie auch Alter und Geschlecht als Kovarianten in den ANOVA Test mit einbezogen. Als Signifikanzlevel wurde 0,05 gesetzt. In der Bestätigungsgruppe wurde der Assoziierungseffekt durch eindimensionales ANOVA mit beiden SNPs zur selben Zeit getestet.

### Ergebnisse

Die Verabreichung von 20 mM Glucose führte in mit Sglt1 mRNA injizierten *Xenopus* Oocyten, nicht jedoch in Oocyten, welche mit Wasser injiziert worden waren, zu einem Einwärtsstrom (I_{glc}) von 48,6 ± 11,5 nA (n = 18). Als Vergleich führte die Glucosebehandlung in mit Wasser injizierten Oocyten zu einem Strom von 1,3 ± 0,7 nA (n = 6). In *Xenopus-*Oocyten, welche mit Sglt1 mRNA und Nedd4-2 mRNA injiziert worden waren (Koexpression), war I_{glc} um 49,2 ± 6,8 % (n = 15) signifikant herabgesetzt. Somit wird Sglt1 durch die Ubiquitinligase Nedd4-2 herunterreguliert (Fig. 1).

Die Koexpression von Wildtyp Sgk1 regulierte den durch Glucose induzierten Strom um 81,3 ± 19.0 % (n = 15) herauf und drehte den Effekt von Nedd4-2 herum. In Oocyten, die Sglt1 zusammen mit Sgk1 und Nedd4-2 exprimierten, war der durch Glucose induzierte Strom um 34,8 ± 11,8 % (n = 14) größer als der Wert, der in Oocyten beobachtet wurde, die Sglt1 allein exprimierten (Fig. 1).

Konstitutiv aktive ^{S422D}Sgk1 war ähnlich effektiv wie Wildtyp Sgk1 (Fig. 2). Die Koexpression von ^{S422D}Sgk1 erhöhte den durch Glucose induzierten Strom um 72,4 ± 9,1 % (n = 57). In dieser Serie von Experimenten erniedrigte die Koexpression von Nedd4-2 den Strom um 35,3 ± 4,4 % (n = 46). Dieser Effekt wurde durch zusätzliche Koexpression von ^{S422D}Sgk1 umgedreht. In Oocyten, die Nedd4-2 und ^{S422D}Sgk1 koexprimierten, war der Strom um 59,2 ± 19,8 % (n = 16) größer als in Oocyten, die Sglt1 allein exprimierten (Fig. 2). Im Gegensatz zu Wildtyp oder konstitutiv aktiver Sgk1 veränderte die inaktive Mutante ^{K127N}Sgk1 den durch Substrat induzierten Strom (- 2,0 ± 5,3 %, n = 14) nicht signifikant und drehte den Effekt von Nedd4-2 nicht um. In Oocyten, die Sglt1 zusammen mit ^{K127N}Sgk1 und Nedd4-2 exprimierten, war der durch Glucose induzierte Strom um 54,9 ± 9,7 % (n = 8) kleiner als der Wert, der in Oocyten beobachtet wurde, die Sglt1 allein exprimierten (Fig. 2).

Der Effekt von Sgk1 wurde durch ^{T308D,S473D}PKB nachgeahmt (Fig. 3). In dieser Serie von Versuchen erniedrigte die Koexpression von Nedd4-2 den Strom um 26,5 ± 5,5 % (n = 42). Die Koexpression mit konstitutiv aktiver ^{T308D,S473D}PKB erhöhte den durch Glucose induzierten Strom in Oocyten, die Sglt1 exprimierten, signifikant um 117,4 ± 15,8 % (n = 31) und drehte den Effekt von Nedd4-2 um. In Xenopus-Oocyten, die ^{T308D,S473D}PKB und Nedd4-2 zusammen mit Sglt1 koexprimierten, war der durch Glucose induzierte Strom um 106,5 ± 18,2 % (n = 27) größer als der Strom in Xenopus-Oocyten, die Sglt1 allein exprimierten (Fig. 3).

Vergleichbar mit ^{T308D,S473D}PKB und Sgk1 stimulierte Sgk3 den durch Glucose induzierten Strom und drehte den Effekt von Nedd4-2 um. Verglichen mit dem durch Glucose induzierten Strom in *Xenopus-*Oocyten, die Sglt1 allein exprimierten, war der durch Glucose induzierte Strom in Oocyten, die Sglt1 und Sgk3 exprimierten, um 123,6 ± 15,0 % ) (n = 22) größer und in Oocyten, die Sglt1, Nedd4-2 und Sgk3 exprimierten, um 112,4 ± 19,4 % (n = 22) größer.

In Fig. 4 ist gezeigt, dass die Koexpression von Sglt1 und ^{S422D}Sgk1 (SD) I_{glc} um 77 ± 23 % auf 65,4 ± 10,6 nA (n = 18) steigert. In Oocyten, welche Sglt1 zusammen mit Sgk1 und Nedd4-2 exprimierten, erreichte der Glucose-induzierte Strom 60,5 ± 9,9 nA (n = 18), das sind 61 ± 21 % mehr als der entsprechende Wert in ausschließlich mit Sglt1 injizierten Oocyten und 126 ± 23% mehr als in Oocyten, welche mit Sglt1 und Nedd4-2 mRNA injiziert worden waren. Bei diesen Experimenten wurde der Strom durch 5 mM Glucose induziert.

In einer weiteren Serie von Experimenten wurden neben der konstitutiv aktiven ^{S422D}Sgk1 (SD) auch die Isoformen der Sgk, Sgk2 und Sgk3, sowie die Proteinkinase B (PKB) getestet. Der Glucose-induzierte Strom wurde durch die Koexpression von ^{S422D}Sgk1 um 55 ± 12 % (n = 44), von Sgk3 um 117 ± 16 % (n = 16) und von PKB um 101 ± 18 % (n = 24) gesteigert, während Sgk2 ohne statistisch signifikante Wirkung blieb. Die Koexpression von Nedd4-2 senkte den Glucosetransport um 23 ± 4 % (n = 79), verhinderte jedoch nicht die Stimulation durch die zusätzliche ) Koexpresion von ^{S422D}Sgk1 (+48 ± 11 %, n = 48), von Sgk3 (+114 ± 26 %, n = 16) und von PKB (+107 ± 20 %, n = 24). Sgk2 blieb wiederum ohne signifikante Wirkung.

Um die funktionale Relevanz von Sgk1 in der Regulation von Sglt1 und Körpergewicht zu untersuchen, wurde der Körpermassen-Index von Zwillingen mit Polymorphismen des sgk1-Gens korreliert. Die durchschnittliche Körpermasse der Zwillinge, die den Polymorphismus E8CC/CT;I6CC trugen, belief sich auf 26.7 ± 1,4 kg/m² (n = 13). Dieser Wert ist signifikant größer (P<0,008) als die entsprechenden Durchschnittswerte (23,3 ± 0,2 kg/m², n = 263) der gesamten Menge der Zwillinge.

Insgesamt zeigen die Experimente einen starken stimulatorischen Effekt von Sgk1, Sgk3 und PKB auf Sglt1. Durch die gesteigerte Sglt1-Aktivität wird die intestinale Glucoseabsorption beschleunigt, so dass die Glucosekonzentration im Plasma schneller ansteigt. Dies bewirkt eine gesteigerte Freisetzung von Insulin und somit eine Stimulierung der Lipidablagerung in Fettgewebe. Andererseits wirken Inhibitoren von Sgit1 einer Fettleibigkeit entgegen.

Die Zwillingsstudien zeigen, dass derselbe Polymorphismus, der mit einem erhöhten Blutdruck assoziiert ist, ebenfalls mit einem höheren Körpermassen-Index zusammenhängt.

## Patentansprüche

1. Verfahren zur in vitro-Diagnose von Prädispositionen für Fettsucht, **dadurch gekennzeichnet, dass** der Polymorphismus E8CC/CT; I6CC in Sgk1 nachgewiesen wird.

2. Diagnosekit, umfassend mindestens eine Substanz zum Nachweis der Expression und/oder Funktion von aktivierter Sgk1 und/oder aktivierter Sgk3 und/oder aktivierter PKB und/oder inaktiver Nedd4-2 zur Diagnose von Fettsucht.

3. Antikörper, **dadurch gekennzeichnet, daß** er gegen mindestens eine phosphorylierte Sgk1-Konsensussequenz im Nedd4-2-Protein gerichtet ist.

4. Antikörper, **dadurch gekennzeichnet, daß** er gegen mindestens eine nicht phosphorylierte Sgk1-Konsensussequenz im Nedd4-2-Protein gerichtet ist.

5. Antikörper, **dadurch gekennzeichnet, daß** er gegen mindestens eine mutierte Sgk1-Konsensussequenz im Nedd4-2-Protein gerichtet ist.

6. Antikörper nach Anspruch 5, **dadurch gekennzeichnet, daß** das Nedd4-2-Protein mit mutierter Sgk1-Konsensussequenz ^{S338D}Nedd4-2 und/oder ^{S444D}Nedd4-2 ist.

## Claims

1. A method for in vitro diagnosis of predispositions to obesity, **characterized in that** the polymorphism E8CC/CT; I6CC in Sgk1 is detected.

2. A diagnostic kit which comprises at least one substance for detecting the expression and/or function of activated Sgk1 and/or activated Sgk3 and/or activated PKB and/or inactive Nedd4-2 for diagnosing obesity.

3. An antibody, **characterized in that** it is directed against at least one phosphorylated Sgk1 consensus sequence in the Nedd4-2 protein.

4. An antibody, **characterized in that** it is directed against at least one unphosphorylated Sgk1 consensus sequence in the Nedd4-2 protein.

5. An antibody, **characterized in that** it is directed against at least one mutated Sgk1 consensus sequence in the Nedd4-2 protein.

6. The antibody as claimed in claim 5, **characterized in that** the Nedd 4-2 protein with a mutated Sgk1 consensus sequence is ^{S338D}Nedd4-2 and/or ^{S444D}Nedd4-2.

## Revendications

1. Procédé pour le diagnostic in vitro de prédispositions à l'obésité, **caractérisé en ce qu'**on détermine le polymorphisme E8CC/CT; I6CC dans Sgkl.

2. Nécessaire de diagnostic, comprenant au moins une substance destinée à la détermination de l'expression et/ou de la fonction de Sgk1 activée et/ou de Sgk3 activée et/ou de PKB activée et/ou de Nedd4-2 inactive, pour le diagnostic de l'obésité.

3. Anticorps, **caractérisé en ce qu'**il est dirigé contre au moins une séquence consensus de Sgk1 phosphorylée dans la protéine Nedd4-2.

4. Anticorps, **caractérisé en ce qu'**il est dirigé contre au moins une séquence consensus de Sgk1 non phosphorylée dans la protéine Nedd4-2.

5. Anticorps, **caractérisé en ce qu'**il est dirigé contre au moins une séquence consensus de Sgk1 mutée dans la protéine Nedd4-2.

6. Anticorps selon la revendication 5, **caractérisé en ce que** la protéine Nedd4-2 à séquence consensus de Sgk1 mutée est ^{S338D}Nedd4-2 et/ou ^{S444D}Nedd4-2.
